# EUROPÄISCHE PATENTSCHRIFT

(11) **EP 1 502 946 B1**
(45) Veröffentlichungstag und Bekanntmachung des Hinweises auf die Patenterteilung: **04.02.2009**
(21) Anmeldenummer: 04018255.2
(22) Anmeldetag: 02.08.2004
(51) Int. Cl.: C12M 1/02, C12M 1/14, C12M 1/16

(54) **Solid State Fermenter**
Solid state fermenter
Fermenteur en millieu solide

(30) Priorität: 31.07.2003 DE 10335522
(43) Veröffentlichungstag der Anmeldung: 02.02.2005
(73) Patentinhaber: PROPHYTA BIOLOGISCHER PFLANZENSCHUTZ GMBH, 23999 Malchow (DE)
(72) Erfinder: Lüth, Peter, 23970 Wismar (DE)
(74) Vertreter: Baumbach, Friedrich

(56) Entgegenhaltungen:
- EP-A- 0 316 910
- WO-A-99/57239
- US-A- 3 575 813
- US-B1- 6 197 573

## Beschreibung

Die Erfindung betrifft einen Solid-State-Fermenter zur Kultivierung von Mikroorganismen an festen Substraten, insbesondere für große Volumina. Anwendungsgebiet der Erfindung ist die mikrobiologische Industrie.

Aus WO 99/57239 ist bereits ein Solid-State-Fermenter bekannt, der aus mindestens zwei luft- und wasserdurchlässigen, übereinander angeordneten Fermenteretagen besteht, die so mit der Wandung des Behälters verbunden sind, daß weder Luft noch Wasser seitlich vorbeiströmen kann, wobei sich auf den Fermenteretagen ein Kultursubstrat für die zu kultivierenden Mikroorganismen befindet und unter jeder Etage eine Kühleinrichtung angebracht ist

Dieser Fermenter weist im Gebrauch mehrere Nachteile auf.
1. Durch die Verwendung eines Kühlsystems, das für jede einzelne Fermenteretage in Form von Kühlschlangen, die unter den Etagen verlaufen und mit jeweils einem Kühlmittelzufluß sowie einem Kühlmittelabfluß durch die Fermenterwand hindurch von außen versorgt werden muß, ergibt sich eine große Gefahr der Kontamination des Fermentergutes mit dem zur Kühlung verwendeten Kühlmittel. Diese Gefahr der Kontamination ist insbesondere dadurch bedingt, daß jede Kühlschlange mit Hilfe von 2 Kupplungen (für Zu- und Abfluß des Kühlmittels) mit den zum Kühlmittelzu- und -abfluß dienenden Rohren außerhalb des Fermenters verbunden werden muß. An diesen Kupplungen kann es zu Leckagen kommen. Die Gefahr des Auftretens solcher Leckagen steigt mit der zunehmenden Anzahl von Fermenteretagen. Durch die von außen seitlich in den Fermenter hineinragenden Anschlußstücke des Kühlsystems (ein Zu- und Abfluß je Fermenteretage) ist die Einbringung der darunter liegenden Fermenteretagen mit Schwierigkeiten verbunden. Sie müssen bei der Einbringung entweder schräg gehalten werden oder mit einer Aussparung versehen sein, um an den Anschlüssen vorbei in den unteren Teil des Fermenters abgesenkt werden zu können.
2. Ein weiteres Problem bei dem unter WO 99/57239 beschriebenen Solid-State-Fermenter besteht darin, daß die Fermenteretagen auf "Ringe oder anders geformte Vorrichtungen" aufgesetzt werden (Anspruch 4). Diese "Ringe oder anders geformte Vorrichtungen" sind mit einer hitzestabilen Dichtung versehen. Damit soll verhindert werden, daß Wasser oder Luft an den Fermenteretagen vorbeifließen kann. Diese "Ringe oder anders geformte Vorrichtungen" bewirken aber, daß die Einbringung der jeweils darunter liegenden Etage mit Schwierigkeiten verbunden ist.

Der Erfindung lag daher die Aufgabe zugrunde, die genannten Nachteile des in WO 99/ 57239 beanspruchten Solid-State-Fermenters durch konstruktive Änderungen zu beseitigen und den Einsatz großer Volumina zu ermöglichen.

Diese Aufgabe wird gemäß der unten beschriebenen Erfindung gelöst. Wesentliche Merkmale der Erfindung sind
- eine neuartige Kühlung des Fermenterguts und
- eine neuartige Abdichtung der Fermenteretagen zur Fermenterwand.

### 1. Kühlung des Fermentergutes

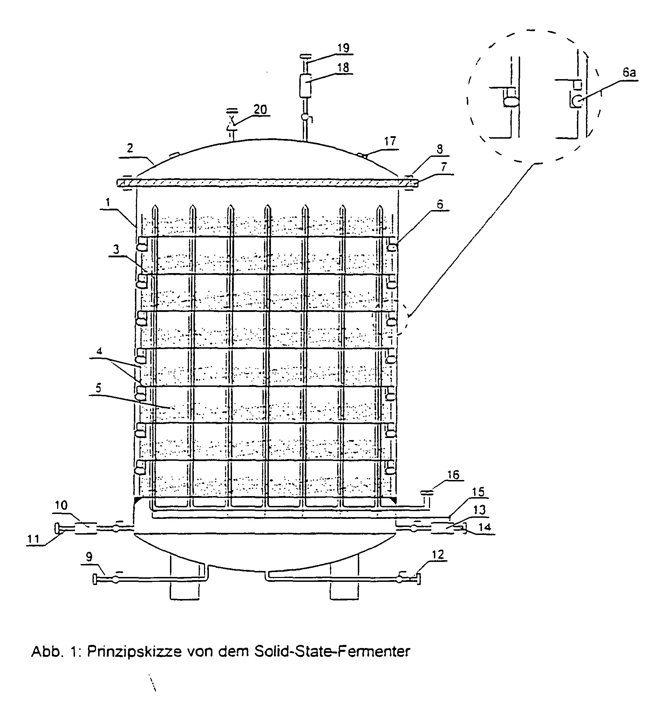

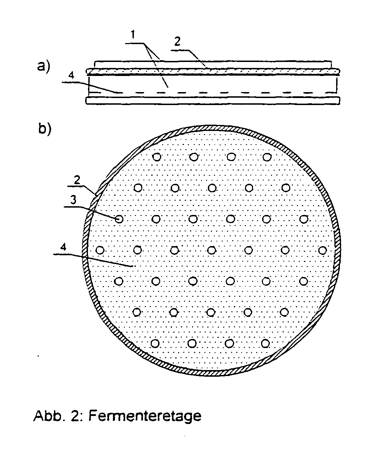

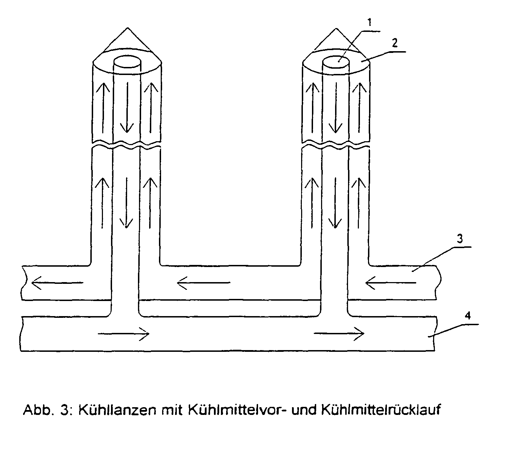

Die Kühlung des Fermentergutes wird durch senkrecht vom Fermenterboden ausgehende Kühllanzen (Abb. 1/3) gewährleistet. Der Abstand der Kühllanzen bestimmt sich durch die Menge der von den kultivierten Mikroorganismen produzierte Wärme. Die Kühllanzen sind in einem triangularen Verbund (Abb. 2) zueinander angeordnet, so daß jede Kühllanze zu den nächstliegenden den gleichen Abstand aufweist. Die Kühllanzen bestehen aus einem innen liegenden Rohr mit geringerem Durchmesser, das dem Rücklauf des Kühlmittels dient, sowie einem Rohr mit größerem Durchmesser, in dem das erste mittig angeordnet ist, das dem Vorlauf des Kühlmittels dient (Abb. 3). Im Querschnitt stellt die Kühllanze einen Kreisring dar. Das Außenrohr ist am oberen Ende mit einem kreiskegelförmigen Verschluß versehen. Das Innenrohr endet offen vorzugsweise 1 - 2 cm vor dem Verschluß des Außenrohres. Die Kühllanzen haben einen Außendurchmesser von vorzugsweise 1 - 3 cm. Das Verhältnis der Durchmesser der beiden Rohre zueinander sollte vorzugsweise so gestaltet werden, daß die Strömungsgeschwindigkeit der Kühlflüssigkeit im Vorlauf gleich der Strömungsgeschwindigkeit der Kühlflüssigkeit im Rücklauf ist.

Die Kühllanzen sind unterhalb der untersten Fermenteretage wie in Abb. 1 dargestellt mit 2 in den Fermenter hineinführenden Rohren verbunden, wobei das eine zum Vorlauf und das andere zum Rücklauf der Kühlflüssigkeit dient. Somit muß die Außenwand des Fermenters, der als Druckgefäß ausgelegt ist, zum Zweck der Kühlung des Fermentergutes lediglich an 2 Stellen durchbrochen werden.

Die Böden der Fermenteretagen sind mit Löchern versehen. Die Löcher haben einen Durchmesser, der um ca. 1 mm größer ist als die Außendurchmesser der Kühllanzetten. Sie können mit Deckeln versehen sein, die sich nach oben hin, also in das Kultursubstrat hinein, öffnen. Beim Einbringen der Fermenteretagen von oben in den Fermenter werden diese Deckel durch die Kühllanzen aufgestoßen und geben somit den Weg für dieselben frei. Durch die kreiskegelförmige Spitze können die Kühllanzetten das Kultursubstrat beim Einbringen der Fermenteretagen in den Fermenter problemlos durchdringen. Aufgrund des durch die Kühllanzetten beanspruchten Volumens darf eine Fermenteretage vor ihrer Einbringung in den Fermenter nur soweit mit Kultursubstrat gefüllt werden, daß dieses nach Einbringung in den Fermenter noch ausreichend viel Platz in der Fermenteretage findet.

Die Befüllung der Fermenteretagen kann jedoch auch erfolgen, nachdem die jeweils betreffende Etage bereits in den Fermenter eingebracht wurde, nämlich derart, daß die Etage sich gerade unterhalb der Fermenteröffnung befindet und die Kühllanzen den Fermenterboden bis zur beabsichtigten Höhe des Kultursubstrates durchdrungen haben. Bei dieser Art der Befüllung sind keine Deckel zum Verschluß der Löcher in den Fermenteretagen erforderlich. Die Etagen werden bis zur beabsichtigten Höhe befüllt und in den Fermenter eingelassen, bis sie auf dem darunter liegenden Fermenterboden aufliegen.

In einem Sonderfall kann der beschriebene Fermenter mit nur einer mit dem Kultursubstrat gefüllten Etage betrieben werden. Dies ist insbesondere dann möglich, wenn das verwendete granulöse Kultursubstrat eine sehr stabile Struktur aufweist und daher keine Gefahr besteht, daß sich das Kultursubstrat während der Sterilisation oder der Fermentation verdichtet oder seine Eigenschaften in einer anderen Weise zuungunsten des Kulturprozesses verändert.

### 2. Abdichtung der Fermenteretagen zur Fermenterwand

Zur Fermentation bzw. Kultur eines aeroben Mikroorganismus ist eine kontinuierliche Zufuhr von Sauerstoff zum Kultursubstrat, an dem der Mikroorganismus sich entwickelt, notwendig.
Daher wird entsprechend der unter WO 99/57239 beanspruchten Erfindung Luft durch das Kultursubstrat hindurchgeleitet. Dies ist jedoch nur möglich, wenn die Fermenteretagen zur Wandung des Fermenters hin abgedichtet sind. Ansonsten würde die Luft aufgrund des geringeren Widerstandes an den Etagen vorbeifließen, und das Kultursubstrat wäre nicht ausreichend mit Sauerstoff versorgt. Ein Abdichtung der Fermenteretagen zur Fermenterwandung hin ist zudem für die Beimpfung des Fermenters erforderlich. Die Beimpfung erfolgt entsprechend der unter WO 99/57239 beanspruchten Erfindung, indem der Fermenter bis über die oberste Fermenteretage hinaus mit sterilem Wasser angestaut wird. Durch eine Öffnung im Deckel wird sodann das Inokulum eingebracht, das sich im Wasser verteilt. Eine gleichmäßige Beimpfung aller Fermenteretagen wird im Anschluß daran dadurch erreicht, daß das Wasser durch einen Bodenabfluß wieder aus dem Fermenter abgelassen wird. Damit werden alle Fermenteretagen gleichmäßig durchströmt und zugleich mit dem Inokulum kontaminiert. Dies ist jedoch auch nur möglich, wenn das Wasser nicht an der Seite an den Fermenteretagen vorbeifließt.

Der luft- und wasserdichte Abschluß wird erfindungsgemäß durch eine Dichtung erreicht, die zwischen den Fermenteretagen installiert ist und durch die Masse der jeweils oben liegenden Etage zusammengedrückt wird (Abb. 1/6 und 1/6a). Die Dichtung besteht aus einem elastischen, hitzestabilen Material (z.B. aus Silikon). Durch das Zusammendrücken der Dichtung dehnt sie sich zur Seite hin aus und wird gegen die Innenwand des Fermenters gepreßt. Dadurch ist der notwendige luft- und wasserdichte Abschluß gewährleistet. Um zu verhindern, daß die Dichtung zu stark zusammengepreßt wird, steht die jeweils oben liegende Fermenteretage auf der darunterliegenden. Dies kann z. B. durch Abstandshalter (Abb. 1/6a) erreicht werden, durch die der Raum für die zusammengepreßte Dichtung zwischen allen Etagen gleich groß ist. Die Dichtung kann entweder am oberen Rand der unteren Etage oder am Rand unter dem Boden der oberen Etage z.B. mit Hilfe einer Nut installiert sein.

Lediglich die unterste Fermenteretage, die zumeist zur Aufnahme eines Befeuchtungsmediums genutzt wird, steht auf einem an der Fermenterwand fest installierten Ring (Abb. 1), auf dem eine Dichtung liegt.

### Bezugszeichenliste

### Abb. 1

- 1: Fermenterwand
- 2: Fermenterdeckel
- 3: Kühllanze
- 4: Fermenteretage
- 5: Substrat zur Kultur der Mikroorganismen
- 6: Dichtung zwischen den Fermenteretagen
- 6a: entspannte Dichtung
- 7: Deckeldichtung
- 8: Deckelverschraubung
- 9: Dampfeinlaß
- 10: Wassersterilfilter
- 11: Wassereinlauf
- 12: Ablauf
- 13: Luftsterilfilter
- 14: Lufteinlaß
- 15: Kühlmittelrücklauf
- 16: Kühlmittelvorlauf
- 17: Inokulationsöffnung
- 18: Abluftsterilfilter
- 19: Luftauslaß
- 20: Sicherheitsventil

### Abb. 2

- a): Vorderansicht
- b): Draufsicht
- 1: Fermeteretagenwand
- 2: Dichtung
- 3: Öffnungen für die Kühllanzen
- 4: Etagenboden, bestehend aus Lochblech

### Abb. 3

- 1: Innenliegendes Rohr
- 2: äußeres Rohr
- 3: Vorlauf
- 4: Rücklauf

## Patentansprüche

1. Solid-State-Fermenter bestehend aus einem oder mehreren luft- und wasserdurchlässigen, übereinander angeordneten Fermenteretagen, die so mit der Wandung des Behälters verbunden sind, daß weder Luft noch Wasser seitlich vorbeiströmen kann, wobei sich auf den Fermenteretagen ein Kultursubstrat für die zu kultivierenden Mikroorganismen befindet,
**dadurch gekennzeichnet, dass**
- die Kühlung des Fermenterguts durch senkrecht vom Fermenterboden ausgehende und durch die Fermenteretagen (4) führende Kühllanzen (3) realisiert wird und
- eine Dichtung (6) zwischen den Fermenteretagen (4) installiert wird, die durch die Masse der jeweils oben liegenden Etage (4) gegen die Innenwand des Fermenters gepreßt wird.

2. Solid-State-Fermenter nach Anspruch 1, **dadurch gekennzeichnet, dass** die Kühllanzen (3) in einem triangularen Verband zueinander angeordnet sind und jede Kühllanze zu den nächstliegenden den gleichen Abstand aufweist.

3. Solid-State-Fermenter nach Anspruch 1 - 2, **dadurch gekennzeichnet, dass** die Kühllanzen (3) aus einem innen liegenden Rohr mit geringem Durchmesser, das dem Rücklauf des Kühlmittels dient, sowie einem Rohr mit größerem Durchmesser, in dem das erste mittig angeordnet ist, das dem Vorlauf des Kühlmittels dient, bestehen.

4. Solid-State-Fermenter nach Anspruch 1 - 3, **dadurch gekennzeichnet, dass** das Außenrohr am oberen Ende mit einem kreiskegelförmigen Verschluß versehen ist und das Innenrohr vorzugsweise 1 - 2 cm vor dem Verschluß des Außenrohres offen endet.

5. Solid-State-Fermenter nach Anspruch 1 - 4, **dadurch gekennzeichnet, dass** die Kühllanzen einen Außendurchmesser von vorzugsweise 1 - 3 cm aufweisen.

6. Solid-State-Fermenter nach Anspruch 1 - 5, **dadurch gekennzeichnet, dass** das Verhältnis der Durchmesser der beiden Rohre zueinander vorzugsweise so gestaltet ist, dass die Strömungsgeschwindigkeit der Kühlflüssigkeit im Vorlauf gleich der Strömungsgeschwindigkeit der Kühlflüssigkeit im Rücklauf ist.

7. Solid-State-Fermenter nach Anspruch 1, **dadurch gekennzeichnet, dass** die Dichtung aus einem elastischen, hitzebeständigen Material besteht, das durch die Masse der jeweils oben liegenden Etage (4) gegen die Innenwand des Fermenters gepreßt wird.

8. Solid-State-Fermenter nach Anspruch 1 und 7, **dadurch gekennzeichnet, dass** die Dichtung aus Silikon besteht.

9. Solid-State-Fermenter nach Anspruch 1 und 7 - 8, **dadurch gekennzeichnet, dass** zwischen den Etagen (4) Abstandshalter (6a) angebracht sind.

10. Solid-State-Fermenter nach Anspruch 1 und 7 - 9, **dadurch gekennzeichnet, dass** die Dichtung entweder am oberen Rand der unteren Etage (4) oder am Rand unter dem Boden der oberen Etage (4) z. B. mit Hilfe einer Nut installiert ist.

11. Solid-State-Fermenter nach Anspruch 1 und 7 - 10, **dadurch gekennzeichnet, dass** die unterste Fermenteretage (4), die zumeist zur Aufnahme eines Befeuchtungsmediums genutzt wird, auf einem an der Fermenterwand fest installierten Ring steht, auf dem eine Dichtung liegt.

## Claims

1. Solid-state fermenter comprising one or more air and water-permeable fermenter levels arranged above one another connected with the walls of the container in such a way that neither air nor water can flow past laterally, with a culture substrate to be found on the fermenter levels for the micro-organisms to be cultivated,
wherein
- the cooling of the fermenter material is achieved by cooling lances (3) vertically rising from the fermenter base and leading through the fermenter levels (4) and
- a seal (6) is installed between the fermenter levels (4), the seal being pressed against the inner wall of the fermenter by the mass of the level (4) above it.

2. Solid-state fermenter according to Claim 1, wherein the cooling lances (3) are positioned in a triangular arrangement to one another and each cooling lance manifests the same distance from the closest one.

3. Solid-state fermenter according to Claims 1 - 2, wherein the cooling lances (3) comprise an internal tube with a lower diameter, which is used for the return of the coolant, and a tube with a larger diameter, in which the first one is arranged centrally and which is used for the feed of the coolant.

4. Solid-state fermenter according to Claims 1 - 3, wherein the outer tube has been provided with a circular cone shaped stopper at the upper end and the inner tube preferably ends open 1 - 2 cm in front of the stopper of the outer tube.

5. Solid-state fermenter according to Claims 1 - 4, wherein the cooling lances preferably manifest an outer diameter of 1 - 3 cm.

6. Solid-state fermenter according to Claims 1 - 5, wherein the ratio of the diameters of the two tubes to one another is preferably designed in such a way that the flow velocity of the coolant in feeding is equal to the flow velocity of the coolant in the return.

7. Solid-state fermenter according to Claim 1, wherein the seal comprises an elastic, heat-resistant material which is pressed against the inner wall of the fermenter by the mass of the level above it (4).

8. Solid-state fermenter according to Claims 1 and 7, wherein the seal comprises silicone.

9. Solid-state fermenter according to Claims 1 and 7 - 8, wherein spacers (6a) have been arranged between the levels (4).

10. Solid-state fermenter according to Claims 1 and 7 - 9, wherein the seal is installed either on the upper edge of the lower level (4) or on the edge below the base of the upper level (4), e.g. with the help of a groove.

11. Solid-state fermenter according to Claims 1 and 7 - 10, wherein the bottom fermenter level (4), which is mainly used to accommodate a moistening medium, is positioned on a ring firmly installed in the fermenter wall, a seal being positioned on the ring.

## Revendications

1. Fermentateur solid-state composé d'un ou de plusieurs étages de fermentation perméables à l'air et à l'eau, placés les uns au-dessus des autres, étant en contact avec la paroi du récipient de sorte que ni air ni eau ne puissent circuler sur les côtés, un substrat de culture pour les microorganismes à cultiver se trouvant sur les étages,
**se caractérisant par le fait que**
- le refroidissement du matériau de fermentation est réalisé par des lances de refroidissement (3) partant à la verticale du fond de fermentation et conduisant au travers des étages de fermentation (4) et
- un joint (6) est installé entre les étages de fermentation (4), joint qui est pressé contre la paroi intérieure du fermentateur par la masse de l'étage respectivement supérieur (4).

2. Fermentateur solid-state selon la revendication 1, **se caractérisant par le fait que** les lances de refroidissement (3) sont placées dans un système triangulaire les unes par rapport aux autres et que chaque lance de refroidissement est à la même distance de celle située le plus près.

3. Fermentateur solid-state selon les revendications 1 - 2, **se caractérisant par le fait que** les lances de refroidissement (3) se composent d'un tube intérieur d'un faible diamètre qui sert au retour du réfrigérant, ainsi que d'un tube d'un diamètre plus grand au milieu duquel le premier tube est placé et qui sert à l'avance du réfrigérant.

4. Fermentateur solid-state selon les revendications 1 - 3, **se caractérisant par le fait que** le tube extérieur est muni à son extrémité supérieure d'un couvercle en forme de cône circulaire et que le tube intérieur prend fin sous forme ouverte de préférence à 1 - 2 cm avant le couvercle du tube extérieur.

5. Fermentateur solid-state selon les revendications 1 - 4, **se caractérisant par le fait que** les lances de refroidissement présentent un diamètre extérieur de 1 - 3 cm de préférence.

6. Fermentateur solid-state selon les revendications 1 - 5, **se caractérisant par le fait que** le rapport des diamètres des deux tubes l'un par rapport à l'autre est de préférence aménagé de sorte que la vitesse d'écoulement du réfrigérant dans l'avance soit la même que la vitesse d'écoulement du réfrigérant dans le retour.

7. Fermentateur solid-state selon la revendication 1, **se caractérisant par le fait que** le joint se compose d'un matériau élastique, résistant à la chaleur et pressé contre la paroi intérieure du fermentateur par la masse de l'étage respectivement supérieur (4).

8. Fermentateur solid-state selon les revendications 1 et 7, **se caractérisant par le fait que** le joint est en silicone.

9. Fermentateur solid-state selon les revendications 1 et 7 - 8, **se caractérisant par le fait que** des écarteurs (6a) sont placés entre les étages (4).

10. Fermentateur solid-state selon les revendications 1 et 7 - 9, **se caractérisant par le fait que** le joint est installé soit au bord supérieur de l'étage inférieur (4) ou au bord sous le fond de l'étage supérieur (4) p. ex. à l'aide d'une rainure.

11. Fermentateur solid-state selon les revendications 1 et 7 - 10, **se caractérisant par le fait que** l'étage de fermentation le plus bas (4) qui est essentiellement utilisé pour la réception d'un milieu d'humidification, se trouve placé sur un anneau fixé à la paroi du fermentateur, anneau sur lequel repose un joint.
